# EUROPEAN PATENT APPLICATION

(11) **EP 4 552 652 A2**
(43) Date of publication of application: **14.05.2025**
(21) Application number: 25165017.2
(22) Date of filing: 15.03.2017
(51) Int. Cl.: A61K 47/38

(54) **TERPENE-ENRICHED CANNABINOID COMPOSITION**

(30) Priority: 16.03.2016 US 201662308961 P
(62) Divisional of application: 17765961.2
(71) Applicant: Buzzelet Development And Technologies Ltd, 4065101 Or-Akiva (IL)
(72) Inventor: EYAL, Aharon M., 93629 16 Levy, Jerusalem (IL); RAZ, Noa, 9974500 Gizo 31, Gizo (IL)
(74) Representative: Harrison IP Limited

(57) **Abstract**

Terpene-enriched cannabinoid composition. Cannabinoid compositions are described including at least one cannabinoid in a specific amount, a primary terpene in a specific amount, at least 5% by weight of a non-cannabinoid, non-terpene, carrier, optionally at least three secondary terpenes; less than 5% by weight glycol; and less than 20% by weight water, where said non-cannabinoid, non-terpene carrier comprises cellulose and the terpenes to cannabinoids weight/weight ratio in said composition is about 0.1 to about 1.0. Also described are the above compositions where said non-cannabinoid, non-terpene carrier comprise less than 5% by weight cellulose and the terpenes to cannabinoids weight/weight ratio in said compositions is about 0.05 to about 1.0, forming terpene-enriched cannabinoid compositions with enhanced therapeutic effect compared with that of a composition comprising the same cannabinoids amounts and one half the amount of said primary terpene.

## Description

### Cross-Reference to Related Applications

This application claims the benefit under 35 U.S.C. § 119(e) of U.S. Provisional Application Ser. No. 62/308,961 filed March 16, 2016, the disclosure of which is expressly incorporated by reference herein in its entirety.

### Technical Field

The field of art to which this invention generally pertains is cannabinoid compositions, and specifically cannabinoid compositions for therapeutic use.

### Background

Terpenes play important roles in cannabinoids-comprising products, affecting the functionality and bioavailability of the cannabinoids and the aroma of the product. Processing cannabis plant material typically leads to terpenes loss so that most of cannabis products are of relatively low terpene content. That is particularly true for cannabis extracts and products thereof, such as cannabis tablets, cannabis gel capsules, cannabis patches, cannabis suppositories, etc. Most cannabis terpenes boiling points are in the range between about 150°C and about 220°C and they evaporate, at least partially, during cannabis buds drying, during solvent separation from extracts and during decarboxylation. Monoterpenes are lost at a rate greater than that of terpenes with a higher molecular weight and terpenes carrying no hydroxyl groups are lost at a rate greater than that of terpenes that do carry hydroxyl groups.

### Summary of the Invention

Provided is a cannabinoid composition comprising (i) at least one cannabinoid in a specific amount, (ii) a primary terpene in a specific amount, (iii) at least 5% by weight of a non-cannabinoid, non-terpene carrier: (iv) optionally at least three secondary terpenes; (v) less than 5% by weight glycol; and (vi) less than 20% by weight water, and (a) wherein said non-cannabinoid, non-terpene carrier comprises cellulose and the terpenes to cannabinoids weight/weight ratio in said composition is about 0.1 to about 1.0, or (b) wherein said non-cannabinoid, non-terpene carrier comprises less than 5% by weight cellulose and the terpenes to cannabinoids weight/weight ratio in said composition is about 0.05 to about 1.0, forming a terpene-enriched cannabinoid composition with enhanced therapeutic effect compared with that of a composition comprising the same cannabinoids amounts and one half the amount of said primary terpene.

According to an embodiment, said enhanced therapeutic effect comprises a shorter onset time, increased magnitude, extended duration, dosages reduction, reduction of secondary adverse symptoms and combinations thereof.

According to an embodiment, said non-cannabinoid, non-terpene, carrier comprises cellulose, and said composition contains (a) tetrahydrocannabinolic acid (THCa) in a concentration of at least 10% by weight; (b) cannabidiolic acid (CBDa) in a concentration of at least 10% by weight; or (c) tetrahydrocannabinolic acid (THCa) in a concentration of at least 5% by weight, and cannabidiolic acid (CBDa) in a concentration of at least 5% by weight; and (i) the water concentration is about 20% to 5% by weight; (ii) said primary terpene forms at least 40% by weight of the total terpene content, and (iii) said primary terpene is selected from the group consisting of pinene, limonene, linalool, caryophyllene, caryophyllene oxide, myrcene, humulene, borneol, eucalyptol, terpineol, nerolidol, phytol, geraniol, bisabolol, camphene, beta-amyrin, thujone, citronellol, pulegone, 1,8-cineole and cycloartenol.

According to an embodiment, said composition comprises (a) tetrahydrocannabinol (THC) in a concentration of at least 10% by weight; (b) tetrahydrocannabinolic acid (THCa) in a concentration of at least 10% by weight; (c) cannabidiol (CBD) in a concentration of at least 10% by weight; (d) cannabidiolic acid (CBDa) in a concentration of at least 10% by weight; or (e) tetrahydrocannabinol (THC) in a concentration of at least 5% by weight, and cannabidiol (CBD) in a concentration of at least 5% by weight; (f) tetrahydrocannabinol acid (THCa) in a concentration of at least 5% by weight, and cannabidiol (CBD) in a concentration of at least 5% by weight; (g) tetrahydrocannabinol acid (THCa) in a concentration of at least 5% by weight, and cannabidiol acid (CBDa) in a concentration of at least 5% by weight; (h) tetrahydrocannabinol (THC) in a concentration of at least 5% by weight, and cannabidiol acid (CBDa) in a concentration of at least 5% by weight, and (i) the non-cannabinoid, non-terpene carrier comprises less than 5% by weight cellulose; (ii) the water concentration is less than 5% by weight; (iii) said primary terpene forms at least 40% by weight of the total terpene content, and (iv) said primary terpene is selected from the group consisting of pinene, limonene, linalool, caryophyllene, caryophyllene oxide, myrcene, humulene, borneol, eucalyptol, terpineol, nerolidol, phytol, geraniol, bisabolol, camphene, beta-amyrin, thujone, citronellol, pulegone, 1,8-cineole and cycloartenol.

According to an embodiment, said primary terpene is selected from the group consisting of pinene, limonene, linalool, caryophyllene, caryophyllene oxide, myrcene, humulene, borneol, eucalyptol, terpineol, nerolidol, phytol, geraniol, bisabolol, camphene, beta-amyrin, thujone, citronellol, pulegone, 1,8-cineole and cycloartenol.

According to an embodiment, the primary terpene and the cannabinoids are present in specific amounts, and the onset time of said therapeutic effect is at least 20% shorter than that of a composition comprising the same cannabinoids amounts and one half the amount of said primary terpene.

According to an embodiment, the primary terpene and the cannabinoids are present in specific amounts, and the magnitude of the therapeutic effect is at least 20% greater compared with that of a composition comprising the same cannabinoids amounts and one half the amount of said primary terpene.

According to an embodiment, the composition comprises THC and/or THCa and/or CBD, and said primary terpene is selected from the group consisting of limonene, linalool, beta-caryophyllene and caryophyllene oxide, resulting in a terpene-enriched cannabinoid composition with enhanced therapeutic effect for treating post trauma syndrome disorder (PTSD). According to an embodiment, the composition includes pinene and/or beta-myrcene, and pinene and/or beta-myrcene form less than 5% by weight of the total terpene content.

According to an embodiment, the composition comprises CBD and optionally THC and/or THCa at CBD to THC and/or THCa weight/weight ratio greater than 1, wherein said primary terpene is selected from the group consisting of limonene, linalool and beta-caryophyllene, resulting in a terpene-enriched cannabinoid composition with enhanced therapeutic effect for treating anxiety. According to an embodiment, the composition includes beta-myrcene, and beta-myrcene form less than 5% by weight of the total terpene content.

According to an embodiment, the composition comprises CBD and optionally THC and/ or THCa at CBD to THC and/or THCa weight/weight ratio greater than 1, and said primary terpene is selected from the group consisting of limonene, linalool, beta-myrcene, and beta-caryophyllene, resulting in a terpene-enriched cannabinoid composition with enhanced therapeutic effect for treating depression.

According to an embodiment, the composition comprises CBD and optionally THC and/ or THCa at CBD to THC and/ or THCa weight/weight ratio greater than 1, and said primary terpene is selected from the group consisting of limonene, linalool, beta-caryophyllene, and piene, resulting in a terpene-enriched cannabinoid composition with enhanced therapeutic effect for treating psychosis syndromes. According to an embodiment, the composition includes beta-myrcene, wherein beta-myrcene form less than 5% by weight of the total terpene content.

According to an embodiment, the composition comprises CBD and optionally THC and/or THCa at CBD to THC and/or THCA weight/weight ratio greater than 1, and said primary terpene is selected from the group consisting of limonene, beta-caryophyllene, caryophyllene oxide, pinene, beta-myrcene, humulene, citronellol, and eucalyptol, resulting in a terpene-enriched cannabinoid composition with enhanced therapeutic effect for treating autism and autism spectrum disorder.

According to an embodiment, the composition comprises CBD and/or THC and/or THCa and said primary terpene is selected from the group consisting of limonene, pinene, linalool, beta-caryophyllene, caryophyllene oxide, pulegone, eucalyptol, terpineol, p-cymene, beta-myrcene, and humulene, resulting in a terpene-enriched cannabinoid composition with enhanced therapeutic effect for treating Alzheimer's disease.

According to an embodiment, the composition comprises CBD and/or THC and/or THCa and said primary terpene is selected from the group consisting of beta-caryophyllene, caryophyllene oxide, beta-myrcene, terpineol, linalool, humulene, eucalyptol, pinene, pulegone, eucalyptol, p-cymene, and limonene, resulting in a terpene-enriched cannabinoid composition with enhanced therapeutic effect for treating Parkinson disease.

According to an embodiment, the composition comprises CBD and/or THC and/or THCa and said primary terpene is selected from the group consisting of beta-caryophyllene, caryophyllene oxide, beta-myrcene, pinene, linalool, limonene, humulene, citronellol, eucalyptol, beta-amyrin, and cycloartenol, resulting in a terpene-enriched cannabinoid composition with enhanced therapeutic effect for treating inflammation, including inflammation from multiple sclerosis, Alzheimer disease, Parkinson disease and traumatic brain injury.

According to an embodiment, the composition comprises CBD and/or THC and/or THCa, and said primary terpene is beta-myrcene or pinene, resulting in a terpene-enriched cannabinoid composition with enhanced therapeutic effect for treating spasticity and muscle tension including spasticity and muscle tension from multiple sclerosis, Parkinson disease, Huntington's disease and Dystonia.

According to an embodiment, the composition comprises CBD and/or THC and/or THCa, and said primary terpene is selected from the group consisting of beta-myrcene, linalool, beta-caryophyllene, humulene, eucalyptol, beta-amyrin, and cycloartenol, resulting in a terpene-enriched cannabinoid composition with enhanced therapeutic effect for treating acute and/or chronic pain including chronic pain from multiple sclerosis, Fibromyalgia, cancer and peripheral neuropathy.

According to an embodiment, the composition comprises CBD and optionally THC and/or THCa at CBD to THC and/or THCA weight/weight ratio greater than 1, and said primary terpene is linalool resulting in a terpene-enriched cannabinoid composition with enhanced therapeutic effect for treating epilepsy.

According to an embodiment, the composition comprises CBD and/or THC and/or THCa, and said primary terpene is selected from the group consisting of beta-myrcene, terpineol, beta-caryophyllene, caryophyllene oxide, pinene, limonene, humulene, citronellol, and eucalyptol, resulting in a terpene-enriched cannabinoid composition with enhanced therapeutic effect for treating stroke and/or traumatic brain injury.

According to an embodiment, the composition comprises THC and/or THCa, and said primary terpene is pinene, resulting in a terpene-enriched cannabinoid composition with enhanced therapeutic effect for treating bronchial disorders including asthma.

According to an embodiment, the composition comprises CBD and/or THC and/or THCa and/or CBDa and said primary terpene is selected from the group consisting of beta-myrcene, limonene, beta caryopyllene, caryopyllene oxide, terpineol, citronellol, linalool, humulene, beta-amyrin, and cycloartenol, resulting in a terpene-enriched cannabinoid composition with enhanced therapeutic effect for treating cancer and cancer related symptoms.

According to an embodiment, the composition comprises CBD and optionally THC and/or THCa at CBD to THC and/or THCA weight/weight ratio greater than 1, and said primary terpene is selected from the group consisting of beta-myrcene, beta- caryopyllene, caryopyllene oxide, and pinene, resulting in a terpene-enriched cannabinoid composition with enhanced therapeutic effect for treating drug abuse.

According to an embodiment, the composition comprises CBD and/or THC and/or THCa and said primary terpene is selected from the group consisting of beta-caryophyllene, caryophyllene oxide, beta-myrcene, terpineol, linalool, and limonene, resulting in a terpene-enriched cannabinoid composition with enhanced therapeutic effect for treating Huntington's disease.

According to an embodiment, the composition comprises CBD and/or THC and/or THCa and said primary terpene is selected from the group consisting of beta-caryophyllene, caryophyllene oxide, and beta-myrcene, resulting in a terpene-enriched cannabinoid composition with enhanced therapeutic effect for treating Dystonia.

According to an embodiment, the composition comprises CBD and/or THC and/or THCa and said primary terpene is selected from the group consisting of beta-caryophyllene, caryophyllene oxide, beta-myrcene, terpineol, linalool humulene, eucalyptol, and limonene, resulting in a terpene-enriched cannabinoid composition with enhanced therapeutic effect for treating Amyotrophic lateral sclerosis (ALS).

According to an embodiment, the composition comprises CBD and/or THC and/or THCa and said primary terpene is selected from the group consisting of beta-myrcene, linalool, and limonene, resulting in a terpene-enriched cannabinoid composition with enhanced therapeutic effect for treating Tourette syndrome.

According to an embodiment, the composition comprises CBD and/or THC and/or THCa and said primary terpene is selected from the group consisting of pinene, terpineol, eucalyptol, pulegone, and p-cymene, resulting in a terpene-enriched cannabinoid composition with enhanced therapeutic effect for treating Myasthenia gravis. According to an embodiment, the composition includes beta-myrcene, and beta-myrcene forms less than 5% by weight of the total terpene content.

According to an embodiment, the composition comprises CBD and optionally THC and/ or THCa at CBD to THC and/or THCa weight/weight ratio greater than 1, and said primary terpene is selected from the group consisting of linalool, beta-myrcene, and beta-caryophyllene, resulting in a terpene-enriched cannabinoid composition with enhanced therapeutic effect for treating sleep disorders.

According to an embodiment, the composition comprises CBD and optionally THC and/ or THCa at CBD to THC and/or THCa weight/weight ratio greater than 1, and said primary terpene is selected from the group consisting of limonene, linalool, and nerolidol, resulting in a terpene-enriched cannabinoid composition with enhanced therapeutic effect for treating skin related syndromes, including acne.

According to an embodiment, the composition additionally contains an additive selected from the group consisting of antioxidants, emulsifiers and texturizers vegetable oils, plant extracts, honey, sucrose, glucose and fructose, pharmaceutical excipients and combinations thereof.

Further provided is a product comprising tablets, gel capsules, medical patches, cigarettes and vaporizer liquids containing said composition.

Provided is a method for producing said composition, comprising providing at least one cannabinoid and blending it with a primary terpene. According to an embodiment, the method, includes extracting cannabis plant material to form an extract. According to an embodiment, extracting comprises contacting said cannabis plant material with an extractant to form an extract, and said extractant comprises at least one terpene.

According to an embodiment, the method comprises synthesizing at least one cannabinoid and blending said synthesized cannabinoid with said primary terpene. According to another embodiment, the method comprises blending cannabis plant material with said primary terpene.

Further provided is a method for treating a patient comprising administering to said patient said composition and/or said product. According to an embodiment, the method comprises (i) administering to said patient for a first period of time a first terpene-enriched cannabis composition comprising a first cannabinoid at a first cannabinoid amount and a first primary terpene at a first primary terpene amount, followed by (ii) administering to said patient for a second period of time a second terpene-enriched cannabis composition comprising said first cannabinoid amount and a second primary terpenes at a second primary terpene amount.

### Detailed Description

Unless indicated otherwise, percent is weight percent and ratio is weight/weight ratio. Unless indicated otherwise, weight ratio means the ratio between weight content, e.g. in an aqueous solution containing 20% solute and 80% water, the solute to water weight ratio is 20:80 or 1:4.

The particulars shown herein are by way of example and for purposes of illustrative discussion of the various embodiments of the present invention only and are presented in the cause of providing what is believed to be the most useful and readily understood description of the principles and conceptual aspects of the invention. In this regard, no attempt is made to show details of the invention in more detail than is necessary for a fundamental understanding of the invention, the description making apparent to those skilled in the art how the several forms of the invention may be embodied in practice.

The present invention will now be described by reference to more detailed embodiments. This invention may, however, be embodied in different forms and should not be construed as limited to the embodiments set forth herein. Rather, these embodiments are provided so that this disclosure will be thorough and complete, and will fully convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for describing particular embodiments only and is not intended to be limiting of the invention. As used in the description of the invention and the appended claims, the singular forms "a," "an," and "the" are intended to include the plural forms as well, unless the context clearly indicates otherwise.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. Every numerical range given throughout this specification will include every narrower numerical range that falls within such broader numerical range, as if such narrower numerical ranges were all expressly written herein.

Additional advantages of the invention will be set forth in part in the description which follows, and in part will be obvious from the description, or may be learned by practice of the invention. It is to be understood that both the foregoing general description and the following detailed description are exemplary and explanatory only and are not restrictive of the invention, as claimed.

Provided is a composition comprising (i) at least one cannabinoid, (ii) a primary terpene and (iii) optionally water at content of less than 20% by weight, wherein primary terpene to cannabinoids weight/weight ratio is greater than 0.05 resulting in a terpene-enriched cannabinoid composition with enhanced therapeutic effect.

Further provided is a cannabinoid composition comprising (i) at least one cannabinoid in a specific amount, (ii) a primary terpene in a specific amount, (iii) at least 5% by weight of a non-cannabinoid, non-terpene, carrier, (iv) optionally at least three secondary terpenes; (v) less than 5% by weight glycol; and (vi) less than 20% by weight water, and (a) wherein said non-cannabinoid, non-terpene carrier comprises cellulose and the terpenes to cannabinoids weight/weight ratio in said composition is about 0.1 to about 1.0 or (b) wherein said non-cannabinoid, non-terpene carrier comprises less than 5% by weight cellulose and the terpenes to cannabinoids weight/weight ratio in said composition is 0.05 to about 1.0, forming a terpene-enriched cannabinoid composition with enhanced therapeutic effect compared with that of a composition comprising the same cannabinoids amounts and one half the amount of said primary terpene.

According to an embodiment, said composition comprises at least two cannabinoids, at least three, at least four or at least five. According to an embodiment, the content of each cannabinoid in said composition is at least 10 parts per million (ppm). As known in the art, cannabinoids have an acid form and a non-acid form (which is also referred to as decarboxylated form, since it can be generated by decarboxylating the acid form). The acid form is indicated herein by the letter (a) at the end of the cannabinoid acronym, e.g. tetrahydrocannabiniolic acid is indicated as THCa, while the decarboxylated form is THC. According to an embodiment, said cannabinoids are selected from the group consisting of tetrahydrocannabiniol in acid or decarboxylated form (THCa or THC, respectively), cannabidiol in acid or decarboxylated form (CBDa or CBD, respectively), cannabigerol in acid or decarboxylated form (CBGa or CBG, respectively), cannabichromene in acid or decarboxylated form (CBCa or CBC, respectively) tetrahydrocannabivarin in acid or decarboxylated form (THCVa or THCV, respectively), Cannabidivarin in acid or decarboxylated form (CBDVa or CBDV respectively) and cannabinol in acid or decarboxylated form (CBNa or CBN, respectively).

According to an embodiment, at least one of said cannabinoids is in acid form. According to an embodiment, at least one of said cannabinoid is at least partially in decarboxylated form. According to an embodiment, at least 50% of said cannabinoid is in decarboxylated form, at least 60%, at least 70%, at least 80% or at least 90%.

According to an embodiment, said composition comprises THC and/or THCa. According to an embodiment, said composition comprises CBD and/or CBDa. According to an embodiment, said composition comprises THC and/or THCa at a content of less than 1%, less than 0.8%, less than 0.6%, less than 0.4% or less than 0.2%. According to an embodiment, said composition comprises both CBD and/or CBDa and THC and/or THCa and the weight/weight ratio between CBD and/or CBDa and THC and/or THCa ((CBD + CBDa)/(THC + THCa)) is at least 10, at least 15, at least 20, at least 25 or at least 30.

According to an embodiment, said composition comprises at least 5% by weight carrier, at least 10%, at least 15%, at least 20%, at least 25%, at least 30%, at least 35% or at least 40% by weight. Any compound other than cannabinoids and terpenes is a suitable carrier. According to an embodiment, said carrier is selected from the group consisting of vegetable oils, e.g. coconut oil, olive oil or sesame oil, pharmaceutical excipients, honey, bees wax, cellulose and combinations thereof. As used herein, the term cellulose refers to cellulose, hemicellulose and their combinations.

According to an embodiment, said composition comprises less than 5% by weight glycol, less than 4%, less than 3%, less than 2%, or less than 1%, by weight glycol. As used herein, the term glycol refers to any glycol, including ethylene glycol, polyethylene glycol, propylene glycol and polypropylene glycol.

According to an embodiment, said composition comprises water and water content is less than 30% by weight, less than 25%, less than 20%, less than 15%, less than 12%, less than 10%, or less than 8%. According to an embodiment, water content is at least 1% by weight, at least 2%, at least 3%, at least 4% or at least 5%.

According to an embodiment, said composition comprises a primary terpene and optionally at least three secondary terpenes, at least four or at least five. The term "terpene", as used herein, refers to both terpenes and terpenoids. As used here, the term "primary terpene" refers to a terpene that forms at least 20% by weight of the total amount of terpenes in the said composition, at least 30%, at least 40%, at least 50%, at least 60%, at least 70% or at least 80%. According to an embodiment, said composition comprises multiple (e.g. two or three) terpenes, each one of which forms at least 20% by weight of the total amount of terpenes in the said composition and each one of these terpenes is considered a primary terpene. As used here, the term "secondary terpene" refers to a terpene that forms at least 10 parts per million (ppm) of said composition. According to an embodiment, the content of said primary terpene in said composition is at least 2 times greater than that of any secondary terpene, at least 4, at least 6, at least 8, at least 10, at least 15, at least 20, at least 25, or at least 30 times greater.

As used herein, "terpenes/cannabinoids (or terpenes to cannabinoids) weight/weight ratio" means the weight ratio between the combined amount of terpenes and the combined amount of cannabinoids. According to an embodiment, said composition comprises at least 5% by weight cellulose and the terpenes to cannabinoids weight/weight ratio in said composition is in the range between about 0.1 and about 1.0. According to an embodiment, said ratio is greater than 0.1, greater than 0.15, greater than 0.2, greater than 0.25, greater than 0.3, greater than 0.35, greater than 0.4, greater than 0.45, greater than 0.5, greater than 0.55, greater than 0.6, greater than 0.65, greater than 0.7, greater than 0.75, greater than 0.8, greater than 0.85, greater than 0.9, greater than 0.95, greater than 1, greater than 1.2, greater than 1.5, greater than 2.0, greater than 3 or greater than 5. According to an embodiment, said ratio is less than 0.9, less than 0.8, less than 0.7, less than 0.6, less than 0.5, less than 0.4, less than 0.3, less than 0.2 or less than 0.15. According to an embodiment, said composition comprising more than 5% cellulose is selected from the group consisting of cannabis plant material, e.g. cannabis buds or cannabis trim, ground forms thereof, plant material preparation for varporizers and cannabis cigarette.

According to another embodiment, said non-cannabinoid, non-terpene carrier comprises less than 5% cellulose and terpenes to cannabinoids weight/weight ratio in said composition is in the range between about 0.05 and about 1.0. According to an embodiment, said ratio is greater than 0.1, greater than 0.15, greater than 0.2, greater than 0.25, greater than 0.3, greater than 0.35, greater than 0.4, greater than 0.45, greater than 0.5, greater than 0.55, greater than 0.6, greater than 0.65, greater than 0.7, greater than 0.75, greater than 0.8, greater than 0.85, greater than 0.9, greater than 0.95, greater than 1, greater than 1.2, greater than 1.5, greater than 2.0, greater than 3 or greater than 5. According to an embodiment, said ratio is less than 0.9, less than 0.8, less than 0.7, less than 0.6, less than 0.5, less than 0.4, less than 0.3, less than 0.2, less than 0.15 or less than 0.1. According to an embodiment, said composition comprising less than 5% cellulose is selected from the group consisting of cannabis trichomes, cannabis extracts and products thereof, such as tablets, gel capsules, medical patches, vaporizer liquids and suppositories.

According to an embodiment, said primary terpene, said secondary terpenes or both are selected from the group consisting of pinene, limonene, linalool, caryophyllene, myrcene, humulene, borneol, eucalyptol, terpineol, nerolidol, phytol, geraniol, bisabolol, camphene, beta-amyrin, thujone, citronellol, pulegone, 1,8-cineole, cycloartenol, isomers thereof and combinations thereof. According to an embodiment, said primary terpene said secondary terpenes or both, are selected from the group consisting of pinene, limonene, linalool, caryophyllene, myrcene, terpineol and combinations thereof. According to an embodiment, at least one of said terpenes is a-cyclic. According to an embodiment, at least one of said terpenes is cyclic. According to an embodiment, at least one of said terpenes is not found in cannabis buds or is present there at less than 0.2%, less than 0.1%, less than 0.05% or less than 0.02%. Such terpene is referred to as "non-cannabis terpene". According to an embodiment, said terpene-enriched cannabinoid composition comprises said non-cannabis terpene at a concentration of at least 0.2%, at least 0.5%, least 0.8%, at least 1%, least 1.5%, at least 2%, at least 3%, least 4%, at least 5%, at least 8% or at least 12%.

According to an embodiment, said primary terpene is selected from the group consisting of pinene, limonene, linalool, caryophyllene, myrcene, humulene, borneol, eucalyptol, terpineol, nerolidol, phytol, geraniol, bisabolol, camphene, beta-amyrin, thujone, citronellol, pulegone, 1,8-cineole, and cycloartenol,. According to an embodiment, said primary terpene is selected from the group consisting of pinene, limonene, linalool, caryophyllene, myrcene and humulene. According to an embodiment, the primary terpene is a non-cannabis terpene.

According to an embodiment, said terpenes comprise at least one monoterpene selected from the group consisting of limonene, myrcene, pinene, linalool, geraniol, terpinolene camphene and isomers thereof. According to an embodiment, said terpenes comprise at least one sesquiterpene selected from the group consisting of nerolidol, caryophyllene, farnesene, zingiberene, vetivazulene, guaiazulene, longifolene, copaene, patchoulol humulene and isomers thereof. According to an embodiment, said terpenes comprise at least one diterpene selected from the group consisting of phytol, retinal, retinol, phytane, cembrene, sclarene, labdane, abietane, texadiene, stemarene, stemoden and isomers thereof. According to an embodiment, said terpenes comprise at least one hydroxy-terpene selected from the group consisting of nerolidol, geraniol, linalool, phytol and isomers thereof. As used herein "hydroxy-terpene" refers to a terpene carrying a hydroxyl function.

According to an embodiment, at least one of said terpenes is a monoterpene, at least one of said terpenes is a sesquiterpene and the monoterpenes to sesquiterpenes weight/weight ratio (i.e. the weight ratio between the total amount of monoterpenes and the total amount of the sesquiterpenes) is greater than 1.5 greater than 2, greater than 2.5, greater than 3, greater than 3.5, greater than 4, greater than 4.5, greater than 5, greater than 6, greater than 7, greater than 8, greater than 9, greater than 10, greater than 15, or greater than 20.

According to an embodiment, at least one of said terpenes is a monoterpene, at least one of said terpenes is a diterpene and the monoterpenes to diterpenes weight/weight ratio (i.e. the weight ratio between the total amount of monoterpenes and the total amount of the diterpenes) is greater than 5, greater than 6, greater than 7, greater than 8, greater than 9, greater than 10, greater than 12, greater than 14, greater than 16, greater than 18, greater than 20, greater than 25, or greater than 30.

According to an embodiment, at least one of the terpenes carries no hydroxyl group, at least one of the terpenes carries hydroxyl group and the non-hydroxy-terpenes to hydroxyl-terpenes weight/weight ratio (i.e. the weight ratio between the total amount of non-hydroxy-terpenes and the total amount of the hydroxy-terpenes) is greater than 1.5, greater than 2, greater than 2.5, greater than 3, greater than 3.5, greater than 4, greater than 4.5, greater than 5, greater than 6, greater than 7, greater than 8, greater than 9, greater than 10, greater than 15, or greater than 20.

According to an embodiment, terpenes form at least 20% by weight of said composition, at least 30%, at least 40%, at least 50%, at least 60% or at least 70%.

According to an embodiment, said composition is liquid while at 30°C. According to an embodiment, said composition is a suspension, while at 30°C. According to an embodiment, said composition is essentially clear of haze or suspended solids, while at 30°C.

According to an embodiment, said composition comprises cannabis plant material. According to an embodiment, said composition comprises cannabis bud. According to an embodiment, said cannabis bud forms at least 20% of said composition, at least 30%, at least 40%, at least 50%, at least 60%, at least 70% or at least 80%.

According to an embodiment, said non-cannabinoid, non-terpene, carrier comprises cellulose, and said composition contains (a) tetrahydrocannabinolic acid (THCa) in a concentration of at least 10% by weight; (b) cannabidiolic acid (CBDa) in a concentration of at least 10% by weight; or (c) tetrahydrocannabinolic acid (THCa) in a concentration of at least 5% by weight, and cannabidiolic acid (CBDa) in a concentration of at least 5% by weight; and (i) the water concentration is between about 20% and 5% by weight; (ii) said primary terpene forms at least 40% by weight of the total terpene content, and (iii) said primary terpene is selected from the group consisting of pinene, limonene, linalool, caryophyllene, caryophyllene oxide, myrcene, humulene, borneol, eucalyptol, terpineol, nerolidol, phytol, geraniol, bisabolol, camphene, beta-amyrin, thujone, citronellol, pulegone, 1,8-cineole and cycloartenol.

According to an embodiment, said non-cannabinoid, non-terpene, carrier comprises cellulose, and said composition contains tetrahydrocannabinolic acid (THCa) and/or cannabidiolic acid (CBDa) in a concentration of at least 10% by weight, at least 12%, at least 14%, at least 16%, at least 18% or at least 20% by weight. According to an embodiment, said composition comprises at least 5% by weight cellulose, at least 10% or at least 15% and water in a concentration of less than 15% by weight, less than 14%, less than 13%, less than 12%, less than 11% or less than 10% by weight. According to an embodiment, said composition comprises at least 5% by weight cellulose and said primary terpenes forms at least 40% by weight of the total terpene content, at least 50%, at least 60%, at least 70%, at least 80% or at least 90% by weight and said primary terpene is selected from the group consisting of pinene, limonene, linalool, caryophyllene, caryophyllene oxide, myrcene humulene, borneol, eucalyptol, terpineol, nerolidol, phytol, geraniol, bisabolol, camphene, beta-amyrin, thujone, citronellol, pulegone, 1,8-cineole and cycloartenol. According to an embodiment, said composition comprises a dried cannabis plant material. According to an embodiment, said composition comprises ground cannabis plant material, is a vaporizer cannabis filling material or is a cannabis cigarette.

According to an embodiment, said non-cannabinoid, non-terpene, carrier comprises cellulose, and said composition contains, tetrahydrocannabinolic acid (THCa) in a concentration of at least 5%wt, at least 6%wt, at least 7%wt, at least 8%wt, at least 9%wt or at least 10%wt and cannabidiolic acid (CBDa) in a concentration of at least 5%wt, at least 6%wt, at least 7%wt, at least 8%wt, at least 9%wt or at least 10%wt. According to an embodiment, said composition comprises at least 5% by weight cellulose and water in a concentration of less than 15% by weight, less than 14%, less than 13%, less than 12%, less than 11% or less than 10% by weight. According to an embodiment, said composition comprises at least 5% by weight cellulose and said primary terpenes forms at least 40% by weight of the total terpene content, at least 50%, at least 60%, at least 70%, at least 80% or at least 90% by weight and said primary terpene is selected from the group consisting of pinene, limonene, linalool, caryophyllene, caryophyllene oxide, myrcene humulene, borneol, eucalyptol, terpineol, nerolidol, phytol, geraniol, bisabolol, camphene, beta-amyrin, thujone, citronellol, pulegone, 1,8-cineole and cycloartenol. According to an embodiment, said composition comprises a dried cannabis plant material. According to an embodiment, said composition comprises ground cannabis plant material, is a vaporizer cannabis filling material or is a cannabis cigarette.

According to an embodiment, said composition comprises (a) tetrahydrocannabinolic (THC) in a concentration of at least 10% by weight; (b) tetrahydrocannabinolic acid (THCa) in a concentration of at least 10% by weight (c) cannabidiol (CBD) in a concentration of at least 10% by weight; (d) cannabidiolic acid (CBDa) in a concentration of at least 10% by weight or (e) tetrahydrocannabinol (THC) in a concentration of at least 5% by weight, and cannabidiol (CBD) in a concentration of at least 5% by weight; (f) tetrahydrocannabinol acid (THCa) in a concentration of at least 5% by weight, and cannabidiol (CBD) in a concentration of at least 5% by weight; (g) tetrahydrocannabinol acid (THCa) in a concentration of at least 5% by weight, and cannabidiol acid (CBDa) in a concentration of at least 5% by weight; (h) tetrahydrocannabinol (THC) in a concentration of at least 5% by weight, and cannabidiol acid (CBDa) in a concentration of at least 5% by weight; wherein (i) the cellulose concentration is less than 5% by weight; (ii) the water concentration is less than 5% by weight; (iii) said primary terpene forms at least 40% by weight of the total terpene content, and (iv) said primary terpene is selected from the group consisting of pinene, limonene, linalool, caryophyllene, caryophyllene oxide, myrcene, humulene, borneol, eucalyptol, terpineol, nerolidol, phytol, geraniol, bisabolol, camphene, beta-amyrin, thujone, citronellol, pulegone, 1,8-cineole and cycloartenol.

According to an embodiment, said composition comprises less than 5%wt cellulose and THC and/or THCa and/or CBD and/or CBDa in a concentration of at least 10% by weight, at least 15%, at least 20%, at least 25%, at least 30%, at least 35%, at least 40%, at least 45% or at least 50% by weight. According to an embodiment, said composition comprises less than 5%wt cellulose and water in a concentration of less than 5% by weight, less than 4%, less than 3%, less than 2%, or less than 1% by weight. According to an embodiment, said composition comprises less than 5% by weight cellulose, and said primary terpenes forms at least 40% by weight of the total terpene content, at least 50%, at least 60%, at least 70%, at least 80% or at least 90% by weight and said primary terpene is selected from the group consisting of pinene, limonene, linalool, caryophyllene, caryophyllene oxide, myrcene, humulene, borneol, eucalyptol, terpineol, nerolidol, phytol, geraniol, bisabolol, camphene, beta-amyrin, thujone, citronellol, pulegone, 1,8-cineole and cycloartenol. According to an embodiment, said composition is liquid. According to an embodiment, said composition is selected from the group consisting of vaporizer cannabis filling liquid, cannabis tablets, cannabis suppositories, cannabis gel capsules, cannabis candies, cannabis drinks and cannabis baked products.

According to an embodiment, said composition comprises less than 5%wt cellulose, THC and/or THCa in a concentration of at least 5% by weight, at least 10%, at least 15%, at least 20%, at least 25%, at least 30% or at least 40% by weight and CBD and/or CBDa in a concentration of at least 5% by weight, at least 10%, at least 15%, at least 20%, at least 25%, at least 30% or at least 40% by weight. According to an embodiment, said composition comprises less than 5%wt cellulose and water in a concentration of less than 5% by weight, less than 4%, less than 3%, less than 2%, or less than 1% by weight. According to an embodiment, said composition comprises less than 5%wt cellulose and said primary terpenes forms at least 40% by weight of the total terpene content, at least 50%, at least 60%, at least 70%, at least 80% or at least 90% by weight and said primary terpene is selected from the group consisting of pinene, limonene, linalool, caryophyllene, caryophyllene oxide, myrcene and humulene. According to an embodiment, said composition is liquid. According to an embodiment, said composition is selected from the group consisting of vaporizer cannabis filling liquid, cannabis tablets, cannabis suppositories, cannabis gel capsules, cannabis candies, cannabis drinks and cannabis baked products.

According to an embodiment, said composition comprises an additive selected from the group consisting of antioxidants, emulsifiers and texturizers vegetable oils, plant extracts, honey, pharmaceutical excipients, sucrose, glucose and fructose, pharmaceutical excipients and combinations thereof. According to an embodiment, said composition comprises a surfactant selected from the group consisting of phospholipids, glycerides, glycolipids and combinations thereof. According to an embodiment, said composition further comprises a food-approved texturizer. According to an embodiment, said composition further comprises at least 10ppm ethanol. According to an embodiment, said composition further comprises at least one of vitamin C, vitamin E, polyunsaturated fatty acids, beeswax and coconut oil. According to an embodiment, said product further comprises a sweetener.

According to an embodiment, said composition is selected from the group consisting of cannabis tablets, cannabis gel capsules, cannabis medical patches, cannabis cigarettes and cannabis vaporizer liquids, cannabis suppositories, cannabis candies, cannabis drinks and cannabis baked products.

According to an embodiment, said composition results in an increased therapeutic effect compared with that of a composition comprising the same cannabinoids amounts and a smaller amount of said primary terpene, e.g. one half of that amount. According to various embodiment, said increased therapeutic effect has various forms, e.g. a shorter onset time, increased magnitude, extended duration, reduced dosages, reduced secondary adverse symptoms, and combinations thereof. According to an embodiment, said increased therapeutic effect comprises a shorter onset time, or differently put an earlier effect, which is important particularly in cases of sublingual and topical delivery and in cases where a rapid effect is desired, as in treating pain. According to an embodiment, said increased therapeutic effect comprises extended duration of the therapeutic effect, for example an extended time of pain relief. According to an embodiment, said increased therapeutic effect comprises increased magnitude of the therapeutic effect, enabling achieving a desired therapeutic effect on administering smaller doses of cannabinoids, saving thereby on cost. According to an embodiment, said increased therapeutic effect comprises using smaller doses of cannabinoids and still achieving at least the same beneficial result. According to an embodiment, said increased therapeutic effect comprises reduction of secondary adverse symptoms, e.g. adverse symptoms of the main illness, of ones of another illness and/or ones related to administered said composition or other drugs.

According to an embodiment, the primary terpene and the cannabinoids are present in specific amounts, and the onset time of said therapeutic effect, as measured by methods known in the art, is at least 20% shorter than that of a composition comprising the same cannabinoids amounts and one half the amount of said primary terpene, at least 30%, at least 40%, at least 50% or at least 60% shorter. According to an embodiment, the primary terpene, secondary terpenes and the cannabinoids are present in specific amounts, and the onset time of said therapeutic effect is at least 20% shorter than that of a composition comprising the same cannabinoids amounts, same secondary terpene amounts and one half the amount of said primary terpene, at least 30%, at least 40%, at least 50% or at least 60% shorter. Shorter onset time, or differently put an earlier effect, is important particularly in cases of sublingual and topical delivery and in cases where a rapid effect is desired, as in treating pain.

According to an embodiment, the primary terpene and the cannabinoids are present in specific amounts, and the onset time of said therapeutic effect is at least 20% longer than that of a composition comprising the same cannabinoids amounts and one half the amount of said primary terpene, at least 30%, at least 40%, at least 50% or at least 60% longer. According to an embodiment, the primary terpene, secondary terpenes and the cannabinoids are present in specific amounts, and the onset time of said therapeutic effect is at least 20% longer than that of a composition comprising the same cannabinoids amounts, same secondary terpene amounts and one half the amount of said primary terpene, at least 30%, at least 40%, at least 50% or at least 60% longer. According to an embodiment, compositions of delayed onset time are used in combination with shorter onset time to reach a sustained release effect.

According to an embodiment, the primary terpene and the cannabinoids are present in specific amounts, and the magnitude of the therapeutic effect, as measured by methods known in the art, is at least 20% greater compared with that of a composition comprising the same cannabinoids amounts and one half the amount of said primary terpene, at least 30%, at least 40%, at least 50% or at least 60% greater. According to an embodiment, the primary terpene, secondary terpenes and the cannabinoids are present in specific amounts, and the magnitude of the therapeutic effect is at least 20% greater than that of a composition comprising the same cannabinoids amounts, same secondary terpene amounts and one half the amount of said primary terpene, at least 30%, at least 40%, at least 50% or at least 60% greater. Without wishing to be limited by any particular theory, such increased magnitude of the therapeutic effect may indicate increased bioavailability. Such increased magnitude enables achieving a desired therapeutic effect on administering smaller doses of cannabinoids, saving thereby on cost.

According to an embodiment, the primary terpene and the cannabinoids are present in specific amounts, and the duration of the therapeutic effect, as measured by methods known in the art, is at least 20% longer compared with that of a composition comprising the same cannabinoids amounts and one half the amount of said primary terpene, at least 30%, at least 40%, at least 50% at least 60%.at least 70%, at least 80%, at least 90% or at least 100% longer. According to an embodiment, the primary terpene, secondary terpenes and the cannabinoids are present in specific amounts, and the duration of the therapeutic effect is at least 20% longer than that of a composition comprising the same cannabinoids amounts, same secondary terpene amounts and one half the amount of said primary terpene, at least 30%, at least 40%, at least 50%, at least 60%.at least 70%, at least 80%, at least 90% or at least 100% longer.

According to an embodiment, said enhanced therapeutic effect is observed in treating at least one condition selected from the group consisting of post trauma syndrome disorder (PTSD), anxiety, depression, psychosis syndromes, autism, autism spectrum disorder, Alzheimer's disease, Parkinson disease, inflammation, including inflammation from multiple sclerosis, spasticity and muscle tension including spasticity and muscle tension from multiple sclerosis, Parkinson disease, Huntington's disease and Dystonia , pain, including pain from multiple sclerosis, Parkinson disease and Alzheimer's disease, epilepsy, stroke, traumatic brain injury, bronchial disorders including asthma, cancer and cancer related symptoms, drug abuse, Huntington's disease, Dystonia, Amyotrophic lateral sclerosis (ALS), Tourette syndrome, Myasthenia gravis, sleep disorders and skin related syndromes, including acne.

According to an embodiment, said therapeutic effect is generated while administering said composition in a vaporizer. According to an embodiment, said therapeutic effect is generated while administering said composition sublingually. According to an embodiment, said therapeutic effect is generated while applying said composition, topically.

According to an embodiment, said composition comprises THC and/or THCa and/or CBD, and said primary terpene is selected from the group consisting of limonene, linalool, beta-caryophyllene, and caryophyllene oxide, resulting in a terpene-enriched cannabinoid composition with enhanced therapeutic effect for treating post trauma syndrome disorder (PTSD). According to an embodiment, said composition further includes pinene and/or beta-myrcene, and pinene and/or beta-myrcene form less than 5% by weight of the total terpene content, less than 4%, less than 3%, less than 2%, less than 1% or less than 0.5% by weight. Without wishing to be limited by any particular theory, said primary terpenes in said composition may enhance therapeutic effect for treating PTSD via reducing anxiety and relieving stress.

According to an embodiment, said composition comprises CBD and optionally THC and/or THCa at CBD to THC and/or THCa weight/weight ratio greater than 1, greater than 2, greater than 3, greater than 4 or greater than 5, and said primary terpene is selected from the group consisting of limonene, linalool, and beta-caryophyllene resulting in a terpene-enriched cannabinoid composition with enhanced therapeutic effect for treating anxiety. According to an embodiment, said composition further includes beta-myrcene, and beta-myrcene form less than 5% by weight of the total terpene content, less than 4%, less than 3%, less than 2%, less than 1% or less than 0.5% by weight. Without wishing to be limited by any particular theory, said primary terpenes in said composition may enhance therapeutic effect for treating anxiety via sedating and relieving stress.

According to an embodiment, said composition comprises CBD and optionally THC and/or THCa at CBD to THC and/or THCa weight/weight ratio greater than 1, greater than 2, greater than 3, greater than 4 or greater than 5, and said primary terpene is selected from the group consisting of limonene, linalool, beta-myrcene, and beta-caryophyllene, resulting in a terpene-enriched cannabinoid composition with enhanced therapeutic effect for treating depression. According to an embodiment, said primary terpene is limonene and/or linalool. Without wishing to be limited by any particular theory, said primary terpenes in said composition may enhance therapeutic effect for treating depression via elevating mood and relieving stress.

According to an embodiment, said composition comprises CBD and optionally THC and/or THCa at CBD to THC and/or THCa weight/weight ratio is greater than 1, greater than 2, greater than 3, greater than 4 or greater than 5, and said primary terpene is selected from the group consisting of limonene, linalool beta-caryophyllene, and pinene, resulting in a terpene-enriched cannabinoid composition with enhanced therapeutic effect for treating psychosis syndromes. According to an embodiment, said primary terpene is limonene and/or linalool. According to an embodiment, said composition further includes beta-myrcene, and beta-myrcene forms less than 5% by weight of the total terpene content, less than 4%, less than 3%, less than 2%, less than 1% or less than 0.5% by weight. Without wishing to be limited by any particular theory, said primary terpenes in said composition may enhance therapeutic effect for treating psychosis syndromes via relieving stress and relaxing.

According to an embodiment, said composition comprises CBD and optionally THC and/or THCa at CBD to THC and/or THCa weight/weight ratio greater than 1, greater than 2, greater than 3, greater than 4 or greater than 5, and said primary terpene is selected from the group consisting of limonene, beta-caryophyllene, caryophyllene oxide, pinene, beta-myrcene, humulene, citronellol, and eucalyptol, resulting in a terpene-enriched cannabinoid composition with enhanced therapeutic effect for treating autism and autism spectrum disorder. According to an embodiment, said primary terpene is selected from the group consisting of beta-caryophyllene, pinene, and beta-myrcene. Without wishing to be limited by any particular theory, said primary terpenes in said composition may enhance therapeutic effect for treating autism and autism spectrum disorder via reducing neuroinflammation and excitotoxicity

According to an embodiment, said composition comprises CBD and/or THC and/or THCa and said primary terpene is selected from the group consisting of limonene, pinene, linalool, beta-caryophyllene, caryophyllene oxide, pulegone, eucalyptol, terpineol, p-cymene, beta-myrcene, and humulene, resulting in a terpene-enriched cannabinoid composition with enhanced therapeutic effect for treating Alzheimer's disease. According to an embodiment, said primary terpene is selected from the group consisting of pinene, terpineol, beta-caryophyllene, and beta-myrcene. Without wishing to be limited by any particular theory, said primary terpenes in said composition may enhance therapeutic effect for treating Alzheimer's disease via improving memory retention and improving neuroprotection thought balancing oxidative stress and reducing excitotoxicity.

According to an embodiment, said composition comprises CBD and/or THC and/or THCa and said primary terpene is selected from the group consisting of beta-caryophyllene, caryophyllene oxide, beta-myrcene, terpineol, linalool, humulene, eucalyptol, pinene, pulegone, eucalyptol, p-cymene, and limonene, resulting in a terpene-enriched cannabinoid composition with enhanced therapeutic effect for treating Parkinson disease. According to an embodiment, said primary terpene is selected from the group consisting of pinene, beta-caryophyllene, and beta-myrcene. Without wishing to be limited by any particular theory, said primary terpenes in said composition may enhance therapeutic effect for treating Parkinson disease via reducing muscle tension and improving neuroprotection thought balancing oxidative stress and reducing excitotoxicity.

According to an embodiment, said composition comprises CBD and/or THC and/or THCa and said primary terpene is selected from the group consisting of beta-caryophyllene, caryophyllene oxide, beta-myrcene, pinene, limonene, humulene, citronellol, eucalyptol, beta-amyrin, and cycloartenol, resulting in a terpene-enriched cannabinoid composition with enhanced therapeutic effect for treating inflammation, including inflammation from multiple sclerosis, Alzheimer's disease, Parkinson's disease and traumatic brain injury According to an embodiment, said primary terpene is selected from the group consisting of pinene, beta-caryophyllene, and beta-myrcene. Without wishing to be limited by any particular theory, said primary terpenes in said composition may enhance therapeutic effect for treating inflammation via suppression of the chronic inflammatory response and/or modulating glial cells.

According to an embodiment, said composition comprises CBD and/or THC and/or THCa, and said primary terpene is beta-myrcene or pinene, resulting in a terpene-enriched cannabinoid composition with enhanced therapeutic effect for treating spasticity and muscle tension including spasticity and muscle tension from_ multiple sclerosis Parkinson disease, Huntington's disease and Dystonia. Without wishing to be limited by any particular theory, said primary terpenes in said composition may enhance therapeutic effect for treating spasticity and muscle tension via intervening with the dopaminergic and cholinergic circuits.

According to an embodiment, said composition comprises CBD and/or THC and/or THCa, and said primary terpene is selected from the group consisting of beta-myrcene, linalool, beta-caryophyllene, humulene, eucalyptol beta-amyrin, and cycloartenol, resulting in a terpene-enriched cannabinoid composition with enhanced therapeutic effect for treating acute and/or chronic pain including acute and/or chronic pain from_multiple sclerosis, Fibromyalgia, cancer and peripheral neuropathy. According to an embodiment, said primary terpene is selected from the group consisting of beta-myrcene, linalool, and beta-caryophyllene. Without wishing to be limited by any particular theory, said primary terpenes in said composition may enhance therapeutic effect for treating acute and/ or chronic pain via modulating nociceptive thresholds, inhibiting release of pro-inflammatory molecules, and displaying synergistic effects with other systems influencing analgesia.

According to an embodiment, said composition comprises CBD and optionally THC and/or THCa at CBD to THC and/or THCa weight/weight ratio is greater than 1, greater than 2, greater than 3, greater than 4 or greater than 5, and said primary terpene is linalool resulting in a terpene-enriched cannabinoid composition with enhanced therapeutic effect for treating epilepsy. Without wishing to be limited by any particular theory, said primary terpenes in said composition may enhance therapeutic effect for treating epilepsy via mediating neuronal balance, reducing excitotoxicity and controlling seizures

According to an embodiment, said composition comprises CBD and/or THC and/or THCa, and said primary terpene is beta-myrcene, terpineol, beta-caryophyllene, caryophyllene oxide, pinene, limonene, humulene, citronellol, and eucalyptol, resulting in a terpene-enriched cannabinoid composition with enhanced therapeutic effect for treating stroke and/or traumatic brain injury. According to an embodiment, said primary terpene is selected from the group consisting of beta-myrcene, terpinolene, beta-caryophyllene, and pinene. Without wishing to be limited by any particular theory, said primary terpenes in said composition may enhance therapeutic effect for treating stroke and/or traumatic brain injury via improving neuroprotection by balancing oxidative stress and reducing excitotoxicity.

According to an embodiment, said composition comprises THC and/or THCa, and said primary terpene is pinene, resulting in a terpene-enriched cannabinoid composition with enhanced therapeutic effect for treating bronchial disorders, including asthma.

According to an embodiment, said composition comprises CBD and/or THC and/or THCa and/or CBDa and said primary terpene is selected from the group consisting of beta-myrcene, limonene, beta caryopyllene, caryopyllene oxide, terpineol, citronellol, linalool, humulene, beta-amyrin, and cycloartenol, resulting in a terpene-enriched cannabinoid composition with enhanced therapeutic effect for treating cancer and cancer related symptoms. According to an embodiment, said primary terpene is selected from the group consisting of beta-myrcene, limonene, and citronellol. Without wishing to be limited by any particular theory, said primary terpenes in said composition may enhance therapeutic effect for treating cancer and cancer related symptoms via immune modulation and aggressive antitumor activity and via reducing nausea and pain and elevating mood.

According to an embodiment, said composition comprises CBD and optionally THC and/or THCa at CBD to THC and/or THCa weight/weight ratio greater than 1, greater than 2, greater than 3, greater than 4 or greater than 5 and said primary terpene is selected from the group consisting of beta-myrcene, beta- caryophyllene, caryophyllene oxide, and pinene resulting in a terpene-enriched cannabinoid composition with enhanced therapeutic effect for treating drug abuse. According to an embodiment, said primary terpene is beta-caryophyllene, and/or Caryophyllene oxide. Without wishing to be limited by any particular theory, said primary terpenes in said composition may enhance therapeutic effect for treating drug abuse via modulating dopaminergic circuits and influencing the reward system.

The composition of Claim 1, comprising CBD and/or THC and/or THCa wherein said primary terpene is selected from the group consisting of beta-caryophyllene caryophyllene oxide, beta-myrcene, terpineol, linalool and limonene, resulting in a terpene-enriched cannabinoid composition with enhanced therapeutic effect for treating Huntington's disease. According to an embodiment, said primary terpene is selected from the group consisting of pinene, beta-caryophyllene, and beta-myrcene. Without wishing to be limited by any particular theory, said primary terpenes in said composition may enhance therapeutic effect for treating Huntington's disease via reducing muscle tension and improving neuroprotection through balancing oxidative stress and reducing excitotoxicity.

The composition of Claim 1, comprising CBD and/or THC and/or THCa wherein said primary terpene is selected from the group consisting of beta-caryophyllene, caryophyllene oxide, and beta-myrcene, resulting in a terpene-enriched cannabinoid composition with enhanced therapeutic effect for treating Dystonia. According to an embodiment, said primary terpene is pinene and/or beta-caryophyllene and/or beta-myrcene. Without wishing to be limited by any particular theory, said primary terpenes in said composition may enhance therapeutic effect for treating Dystonia via reducing muscle tension.

The composition of Claim 1, comprising CBD and/or THC and/or THCa wherein said primary terpene is selected from the group consisting of beta-caryophyllene, caryophyllene oxide, beta-myrcene, terpineol, linalool humulene, eucalyptol, and limonene, resulting in a terpene-enriched cannabinoid composition with enhanced therapeutic effect for treating Amyotrophic lateral sclerosis (ALS). According to an embodiment, said primary terpene is selected from the group consisting of pinene, beta-caryophyllene and beta-myrcene. Without wishing to be limited by any particular theory, said primary terpenes in said composition may enhance therapeutic effect for treating ALS via reducing muscle tension and improving neuroprotection through balancing oxidative stress and reducing excitotoxicity.

The composition of Claim 1, comprising CBD and/or THC and/or THCa wherein said primary terpene is selected from the group consisting of beta-myrcene, linalool, and limonene, resulting in a terpene-enriched cannabinoid composition with enhanced therapeutic effect for treating Tourette syndrome.. Without wishing to be limited by any particular theory, said primary terpenes in said composition may enhance therapeutic effect for treating Tourette syndrome via reducing muscle tension and reducing anxiety.

The composition of Claim 1, comprising CBD and/or THC and/or THCa wherein said primary terpene is selected from the group consisting of pinene, terpineol, eucalyptol, pulegone, and p-cymene, resulting in a terpene-enriched cannabinoid composition with enhanced therapeutic effect for treating Myasthenia gravis. According to an embodiment, said composition further includes beta-myrcene, and beta-myrcene forms less than 5% by weight of the total terpene content, less than 4%, less than 3%, less than 2%, less than 1% or less than 0.5% by weight. Without wishing to be limited by any particular theory, said primary terpenes in said composition may enhance therapeutic effect for treating Myasthenia gravis via reducing muscle tension and intervening with the cholinergic circuits.

According to an embodiment, said composition comprises CBD and optionally THC and/or THCa at CBD to THC and/or THCa weight/weight ratio greater than 1, greater than 2, greater than 3, greater than 4 or greater than 5 and said primary terpene is selected from the group consisting of linalool, beta-myrcene, and beta-caryophyllene, resulting in a terpene-enriched cannabinoid composition with enhanced therapeutic effect for treating sleep disorders. According to an embodiment, said primary terpene is linalool and/or beta-myrcene. Without wishing to be limited by any particular theory, said primary terpenes in said composition may enhance therapeutic effect for treating sleep disorders via relieving stress, relaxation and sedation.

According to an embodiment, said composition comprises CBD and optionally THC and/or THCa at CBD to THC and/or THCa weight/weight ratio greater than 1, greater than 2, greater than 3, greater than 4 or greater than 5 and said primary terpene is selected from the group consisting of limonene, linalool, and nerolidol, resulting in a terpene-enriched cannabinoid composition with enhanced therapeutic effect for treating skin-related syndromes, including acne. Without wishing to be limited by any particular theory, said primary terpenes in said composition may enhance therapeutic effect for treating skin-related syndromes via enhancing skin permeability and entry of other drugs, increasing antibiotic benefits and via inhibiting related bacteria.

According to an embodiment, said terpene-enriched cannabinoid composition of improved therapeutic effect is a humans medication. According to an embodiment, said terpene-enriched cannabinoid composition of improved therapeutic effect is a veterinary medication.

According to an embodiment, the shelf life of said composition is at least 6 months or at least a year. According to an embodiment, primary terpene degradation in said composition is less than 20% per year.

According to an embodiment, further provided is a product comprising tablets, gel capsules, suppositories, energy drinks, bakery products, medical patches, cigarettes and vaporizer liquids containing said composition.

According to an embodiment, further provided is a commercial product comprising two compositions according to the present invention, which two compositions differ in the content of said primary terpene. According to an embodiment, further provided is a commercial product comprising two compositions according to the present invention, which two compositions comprise different primary terpenes.

According to an embodiment, further provided is a method for producing said composition comprising providing at least one cannabinoid and blending it with a primary terpene.

According to an embodiment, said method includes extracting cannabis plant material to form an extract. According to an embodiment said extracting comprises steam distillation. According to an embodiment, said method further comprises removing terpenes from said extract prior to said blending. According to an embodiment, said extracting comprises contacting with an extractant to form an extract, which extract comprises at least one cannabinoid and said extractant; and optionally removing at least a fraction of said extractant from said extract. According to an embodiment, said extractant comprises at least one of ethanol, a liquefied gas, such as butan, butane or dimethyl-ether, liquefied CO₂, near-critical CO₂ supercritical CO₂ and combinations thereof.. According to an embodiment, extracting comprises contacting said cannabis plant material with an extractant to form an extract, and said extractant comprises at least one terpene. According to an embodiment, said extractant comprises said primary terpene.

According to an embodiment, said method further comprises at least partially decarboxylating said cannabinoid. According to an embodiment said decarboxylating is conducted at a temperature greater than 100°C. According to an embodiment said decarboxylating is conducted prior to extracting. According to an embodiment said decarboxylating is conducted on the extract prior to said blending with said primary terpene. According to an embodiment, a fraction of said extract is decarboxylated, and said decarboxylated fraction is blended with another fraction, which was not decarboxylated.

According to an embodiment, said method further comprises extracting a plant material, whereby said primary terpene is produced. According to an embodiment, said plant material is selected from the group consisting of cannabis, lemons, oranges, hops, lavender, pine needles, Echinacea, tea, clover and capsicum.

According to an embodiment, said method comprises synthesizing at least one cannabinoid and blending said synthesized cannabinoid with said primary terpene.

According to an embodiment, said method further comprises blending cannabis plant material with said primary terpene. According to an embodiment, said cannabis plant material comprises a cannabis bud. According to an embodiment, said blending cannabis plant material with said primary terpene comprises spraying said cannabis plant material with said primary terpene, optionally in a solvent. According to an embodiment, said blending cannabis plant material with said primary terpene comprises combining said cannabis plant material with a substrate comprising said primary terpene. According to an embodiment, said substrate is a plant material sprayed with said primary terpene. According to an embodiment, said substrate is a cigarette paper sprayed with said primary terpene.

According to an embodiment, further provided is a method for producing said composition comprising extracting cannabis plant material to form an extract, wherein said extracting forms at least two extract fractions, a cannabinoid-enriched extract and a terpene-enriched extract. As used herein, the term "cannabinoid-enriched extract" refers to an extract wherein cannabinoid to terpenes weight/weight ratio is greater than that in said plant material. As used herein, the term "terpene-enriched extract" refers to an extract wherein cannabinoid to terpenes weight/weight ratio is smaller than that in said plant material. According to an embodiment, said method comprises dividing said cannabinoid-enriched extract into at least two fractions and mixing at least one fraction with at least part of said terpene-enriched extract to form a terpene-enriched composition, wherein terpenes to cannabinoid weight/weight ratio is greater than that ratio in the cannabis plant material. According to an embodiment said ratio is 1.5 times greater than that in the cannabis plant material, 2 times greater, 2.5 times greater, 3 times greater, 4 times greater, 5times greater, 6 times greater, 7 times greater, 8 times greater, or 10 times greater. According to an embodiment, said terpene-enriched composition is further mixed with at least one terpene.

According to an embodiment, further provided is a method for treating a patient comprising administering to said patient said composition. According to an embodiment, said composition is administered in a form selected from the group consisting of cigarettes, vaporizer plant material, vaporizer liquid, extract, tablets, gel capsules, suppositories and combinations thereof. According to an embodiment, the daily dose of said composition comprises about 1 milligram cannabinoid to about 300 milligram, about 2 milligram cannabinoid to about 200 milligram or about 3 milligram cannabinoid to about 100 milligram. According to another embodiment, the daily dose of said composition comprises about 0.5 milligram cannabinoid per kilogram body weight to about 30 milligram cannabinoid per kilogram body weight.

According to an embodiment, said method for treating a patient comprises (i) administering to said patient for a first period of time a first terpene-enriched cannabis composition comprising a first cannabinoid at a first cannabinoid amount and a first primary terpene at a first primary terpene amount, followed by (ii) administering to said patient for a second period of time a second terpene-enriched, cannabis composition comprising said first cannabinoid amount and a second primary terpenes at a second primary terpene amount. According to an embodiment, said method further comprises administering to said patient for a third period of time said first terpene-enriched cannabis composition comprising said first cannabinoid amount and a third primary terpene at a third primary terpene amount. According to an embodiment, said third primary terpene is identical to said first primary terpene.

According to another embodiment, said method for treating a patient comprises (i) administering to said patient a first terpene-enriched cannabis composition comprising a first cannabinoid at a first cannabinoid amount and a first primary terpene at a first primary terpene amount and administering to said patient, at least 2 hours later a second terpene-enriched cannabis composition comprising said first cannabinoid amount and a second primary terpenes at a second primary terpene amount. According to another embodiment, said first terpene-enriched cannabis composition is administered for day time and said second terpene-enriched cannabis composition is administered for night time.

According to another embodiment, said method for treating a patient comprises administering to said patient (i) a first terpene-enriched cannabis composition comprising a first cannabinoid at a first cannabinoid amount and a first primary terpene at a first primary terpene amount and (ii) a second terpene-enriched cannabis composition comprising said first cannabinoid and a second primary terpenes at a second primary terpene amount.

According to an embodiment, said method for treating a patient comprises (i) administering to said patient multiple cannabis compositions to find the best working one; (ii) mimicking said best working composition by extracting cannabis plant material to form an extract and blending said extract with suitable terpenes, whereby a mimicking composition is formed and (iii) administering to said patient said mimicking composition. According to an embodiment, said method further comprises removing terpenes from said extract prior to said blending.

### Examples 1- 49

The Table presents examples of compositions as described herein.

| | Form | Cannabinoid | Primary terpene | Content (% by weight) | | Therapeutic effect |
|---|---|---|---|---|---|---|
| | | | | Cannabinoid | Primary terpene | |
| 1 | Cigarette | THC | Limonene | 5-25 | 1-10 | PTSD |
| 2 | Oil | THC plus CBD | Beta-myrcene | 10-40 plus 10-40 | 2-20 | Parkinson disease |
| 3 | Grinded bud | THC | Beta-myrcene | 5-25 | 1-10 | ALS |
| 4 | tablets | THC plus CBD | beta-Caryophyllene | 5-25 plus 5-25 | 1-10 | Dystonia |
| 5 | Cigarette | THCa plus CBD | Limonene | 5-25 plus 5-25 | 1-10 | Anxiety |
| 6 | Grinded bud | THC plus CBD | Limonene | 5-25 plus 5-25 | 1-10 | Depression |
| 7 | tablets | THCa plus CBD | beta-Caryophyllene | 5-25 plus 5-25 | 1-10 | Alzheimer disease |
| 8 | Extract | THC plus CBD | Pinene | 10-40 plus 10-40 | 2-20 | Tourette syndrome |
| 9 | Grinded bud | THC | Pinene | 5-25 | 1-10 | Myasthenia gravis |
| 10 | Grinded bud | THCa plus CBD | Limonene | 5-25 plus 5-25 | 1-10 | Psychosis syndromes |
| 11 | Oil | CBD | Linalool | 10-40 | 2-20 | Depression |
| 12 | Grinded bud | THC | Pinene | 5-25 | 1-10 | Alzheimer disease |
| 13 | Oil | CBD | Linalool | 10-40 | 2-20 | Psychosis syndromes |
| 14 | tablets | THCa plus CBD | beta-Caryophyllene | 5-25 plus 5-25 | 1-10 | Autism and autism spectrum disorder |
| 15 | Grinded bud | THC | Pinene | 5-25 | 1-10 | Parkinson disease |
| 16 | Cigarette | THC | Pinene | 5-25 | 1-10 | Huntington's disease |
| 17 | Oil | THCa plus CBD | beta-Caryophyllene | 10-40 plus 10-40 | 2-20 | Huntington's disease |
| 18 | tablets | THC plus CBD | beta-Caryophyllene | 5-25 plus 5-25 | 1-10 | Inflammation |
| 19 | Extract | THCa plus CBD | Pinene | 10-40 plus 10-40 | 2-20 | Inflammation |
| 20 | Cigarette | THC | Beta-myrcene | 5-25 | 1-10 | Spasticity |
| 21 | Oil | THC plus CBD | Linalool | 10-40 plus 10-40 | 2-20 | Pain |
| 22 | Oil | THCa plus CBD | Linalool | 10-40 plus 10-40 | 2-20 | Epilepsy |
| 24 | tablets | THCa plus CBD | beta-Caryophyllene | 5-25 plus 5-25 | 1-10 | Parkinson disease |
| 25 | tablets | THC plus CBD | Beta-myrcene | 5-25 plus 5-25 | 1-10 | Huntington's disease |
| 26 | Cigarette | THC | Beta-myrcene | 5-25 | 1-10 | Dystonia |
| 27 | Extract | THCa plus CBD | Linalool | 10-40 plus 10-40 | 2-20 | PTSD |
| 28 | Extract | THC plus CBD | Pinene | 10-40 | 2-20 | Dystonia |
| 29 | Oil | THCa plus CBDa | Terpineol | 10-40 plus 10-40 | 2-20 | Cancer |
| 30 | tablets | THC plus CBD | beta-Caryophyllene | 5-25 plus 5-25 | 1-10 | ALS |
| 31 | Oil | THCa plus CBD | Pinene | 10-40 plus 10-40 | 2-20 | ALS |
| 32 | Cigarette | THC | Beta-myrcene | 5-25 | 1-10 | Tourette syndrome |
| 33 | Extract | CBD | Pinene | 10-40 | 2-20 | Autism and autism spectrum disorder |
| 34 | Oil | THCa plus CBD | beta-Caryophyllene | 10-40 plus 10-40 | 2-20 | Autism and autism spectrum disorder |
| 35 | tablets | THC plus CBD | beta-Caryophyllene | 5-25 plus 5-25 | 1-10 | Stroke and/or traumatic brain injury |
| 36 | Extract | THCa plus CBD | Terpineol | 10-40 plus 10-40 | 2-20 | Stroke and/or traumatic brain injury |
| 37 | Oil | THC plus CBD | Beta-myrcene | 10-40 plus 10-40 | 2-20 | Alzheimer disease |
| 38 | Extract | CBD | Citronellol | 10-40 | 2-20 | Cancer |
| 39 | Cigarette | THC plus CBD | Beta-myrcene | 5-25 plus 5-25 | 1-10 | Cancer |
| 40 | Extract | THC plus CBD | Pinene | 10-40 plus 10-40 | 2-20 | Spasticity |
| 41 | Cigarette | THC | Beta-myrcene | 5-25 | 1-10 | Pain |
| 42 | Cigarette | THC | Beta-myrcene | 5-25 | 1-10 | Inflammation |
| 43 | Oil | CBD | Caryopyllene oxide | 10-40 | 2-20 | drug abuse |
| 44 | Extract | THC | Pinene | 10-40 | 2-20 | Asthma |
| 45 | Grinded bud | THC plus CBD | Beta-myrcene | 5-25 plus 5-25 | 1-10 | Stroke and/or traumatic brain injury |
| 46 | Oil | THC plus CBD | Terpineol | 10-40 plus 10-40 | 2-20 | Myasthenia gravis |
| 47 | Cigarette | THC plus CBD | beta-Caryophyllene | 5-25 plus 5-25 | 1-10 | drug abuse |
| 48 | Oil | THC plus CBD | beta-Caryophyllene | 10-40 plus 10-40 | 2-20 | PTSD |
| 49 | Oil | CBD | Linalool | 10-40 | 2-20 | Anxiety |
| | | | | | | |
| | | | | | | |
| | | | | | | |

Thus, the scope of the invention shall include all modifications and variations that may fall within the scope of the attached claims. Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A cannabinoid composition comprising
(i) at least one cannabinoid ;
(ii) a total primary terpene content forming at least 40% by weight of the total amount of terpenes in the composition;
(iii) at least 5% by weight of a non-cannabinoid, non-terpene carrier
(iv) less than 5% by weight glycol; and
(v) less than 20% by weight water,
wherein a terpenes to cannabinoids weight/weight ratio in said composition is about 0.1 to about 1.0.

2. The composition of claim 1, wherein said composition comprises greater than 5% by weight cellulose and at least 50% by weight of a material selected from the group consisting of cannabis buds, cannabis trim or ground forms thereof.

3. The composition of claim 1 or claim 2, wherein said at least one cannabinoid is selected from the group consisting of tetrahydrocannabinol (THC), tetrahydrocannabinolic acid (THCa), cannabidiol (CBD), cannabidiolic acid (CBDa) and combinations thereof.

4. The composition of any one of the preceding claims, wherein said primary terpene is selected from the group consisting of pinene, limonene, linalool, caryophyllene, caryophyllene oxide, myrcene, humulene, borneol, eucalyptol, terpineol, nerolidol, phytol, geraniol, bisabolol, camphene, beta-amyrin, thujone, citronellol, pulegone, 1,8-cineole and cycloartenol and combinations thereof.

5. The composition of any one of the preceding claims, provided in a form selected from the group consisting of cigarettes, vaporizer filling material and vaporizer liquids.

6. The composition of any one of claims 1 to 5, for use in treating a condition selected from the group consisting of post-traumatic stress disorder (PTSD), psychosis syndromes, Alzheimer's disease, Parkinson's disease, inflammation, spasticity and muscle tension, epilepsy, stroke and/or traumatic brain injury, bronchial disorders, cancer, drug abuse, Huntingdon's disease, dystonia, amyotrophic lateral sclerosis, Tourette's syndrome, myasthenia gravis and skin disorders.

7. The composition of any one of claims 2 to 5, for use in treating a condition selected from the group consisting of pain, anxiety, depression, sleep disorders, autism or autism spectrum disorder, post-traumatic stress disorder (PTSD), psychosis syndromes, Alzheimer's disease, Parkinson's disease, inflammation, spasticity and muscle tension, epilepsy, stroke and/or traumatic brain injury, bronchial disorders, cancer, drug abuse, Huntingdon's disease, dystonia, amyotrophic lateral sclerosis, Tourette's syndrome, myasthenia gravis and skin disorders.

8. The composition for use according to claim 7, when said condition is selected from the group consisting of pain, anxiety, depression, a sleep disorder, autism and autism spectrum disorder (ASD), wherein
a) when said condition is selected from the group consisting of acute pain and chronic pain, said at least one cannabinoid is selected from the group consisting of THC, THCa, CBD and combinations thereof, and said primary terpene is selected from the group consisting of beta-myrcene, linalool, beta-caryophyllene, humulene, eucalyptol, beta-amyrin, and cycloartenol;
b) when said condition comprises anxiety, said at least one cannabinoid comprises CBD, and said primary terpene is selected from the group consisting of limonene, linalool and beta-caryophyllene;
c) when said condition comprises depression, said at least one cannabinoid comprises CBD, and said primary terpene is selected from the group consisting of limonene, linalool, beta-myrcene and beta-caryophyllene;
d) when said condition comprises a sleep disorder, said at least one cannabinoid comprises CBD, and said primary terpene is selected from the group consisting of linalool, beta-myrcene, and beta-caryophyllene; and
e) when said condition comprises autism or autism spectrum disorder, said at least one cannabinoid comprises CBD, and said primary terpene is selected from the group consisting of limonene, beta-caryophyllene, caryophyllene oxide, pinene, beta- myrcene, humulene, citronellol, and eucalyptol.

9. The composition for use according to claim 6, wherein said condition is selected from the group consisting of post trauma syndrome disorder (PTSD) and psychosis syndrome, wherein
a) when said condition comprises PTSD, said at least one cannabinoid is selected from the group consisting of THC, THCa, CBD and combinations thereof, and said primary terpene is selected from the group consisting of limonene, linalool, beta-caryophyllene and caryophyllene oxide; and
b) when said condition comprises psychosis syndrome, said at least one cannabinoid comprises CBD, and said primary terpene is selected from the group consisting of limonene, beta-caryophyllene and pinene.

10. The composition for use according to claim 6, wherein said condition is selected from the group consisting of Alzheimer's disease, Parkinson's disease, and Huntingdon's disease, wherein
a) when said condition comprises Alzheimer's disease, said at least one cannabinoid is selected from the group consisting of THC, THCa, CBD and combinations thereof, and said primary terpene is selected from the group consisting of limonene, pinene, linalool, beta-caryophyllene, caryophyllene oxide, pulegone, eucalyptol, terpineol, p-cymene, beta-myrcene, and humulene;
b) when said condition comprises Parkinson's disease, said at least one cannabinoid is selected from the group consisting of THC, THCa, CBD and combinations thereof, and said primary terpene is selected from the group consisting of beta-caryophyllene, caryophyllene oxide, beta- myrcene, terpineol, linalool, humulene, eucalyptol, pinene, pulegone, eucalyptol, p-cymene, and limonene; and
c) when said condition comprises Huntingdon's disease, said at least one cannabinoid is selected from the group consisting of CBD, THC, THCa and combinations thereof and said primary terpene is selected from the group consisting of beta-caryophyllene, caryophyllene oxide, beta- myrcene, terpineol, linalool, and limonene.

11. The composition for use according to claim 6, wherein said condition is selected from the group consisting of inflammation, bronchial disorder and cancer, wherein
a) when said condition comprises inflammation, said at least one cannabinoid is selected from the group consisting of THC, THCa, CBD and combinations thereof, and said primary terpene is selected from the group consisting of beta-caryophyllene, caryophyllene oxide, beta-myrcene, pinene, linalool, limonene, humulene, citronellol, eucalyptol, beta-amyrin, and cycloartenol, and wherein said condition comprises inflammation;
b) when said condition comprises a bronchial disorder, said at least one cannabinoid is selected from the group consisting of THC and THCa and combinations thereof, and said primary terpene comprises pinene; and
c) when said condition comprises cancer, said at least one cannabinoid is selected from the group consisting of CBD, THC, THCa and CBDa, and said primary terpene is selected from the group consisting of beta-myrcene, limonene, beta caryophyllene, caryophyllene oxide, terpineol, citronellol, linalool, humulene, beta-amyrin, and cycloartenol.

12. The composition for use according to claim 6, wherein said condition is selected from the group consisting of spasticity, muscle tension, epilepsy and dystonia, wherein
a) when said condition comprises spasticity and/or muscle tension, said at least one cannabinoid is selected from the group consisting of THC, THCa, CBD and combinations thereof, and said primary terpene is selected from the group consisting of beta-myrcene and pinene;
b) when said condition comprises epilepsy, said at least one cannabinoid comprises CBD, and said primary terpene comprises linalool; and
c) when said condition comprises dystonia, said at least one cannabinoid is selected from the group consisting of CBD, THC, THCa and combinations thereof, and said primary terpene is selected from the group consisting of beta-caryophyllene, caryophyllene oxide, and beta-myrcene.

13. The composition for use according to claim 6, wherein said condition is selected from the group consisting of stroke, traumatic brain injury and Amyotrophic lateral sclerosis (ALS), wherein
a) when said condition is selected from the group consisting of stroke and traumatic brain injury, said at least one cannabinoid is selected from the group consisting of THC, THCa, CBD and combinations thereof, and said primary terpene is selected from the group consisting of beta-myrcene, terpineol, beta-caryophyllene, caryophyllene oxide, pinene, limonene, humulene, citronellol, and eucalyptol; and
b) when said condition comprised ALS, said at least one cannabinoid is selected from the group consisting of THC, THCa, CBD and combinations thereof, and said primary terpene is selected from the group consisting of beta-caryophyllene, caryophyllene oxide, beta- myrcene, terpineol, linalool humulene, eucalyptol, and limonene.

14. The composition for use according to claim 6, wherein said at least one cannabinoid comprises CBD, wherein said primary terpene is selected from the group consisting of beta-myrcene, beta- caryophyllene, caryophyllene oxide, and pinene, and wherein said condition comprises drug abuse.

15. The composition for use according to claim 6, wherein said condition is selected from the group consisting of Tourette syndrome and myasthenia gravis, wherein
a) when said condition comprises Tourette syndrome, said at least one cannabinoid is selected from the group consisting of CBD, THC, THCa and combinations thereof, and said primary terpene is selected from the group consisting of beta-myrcene, linalool, and limonene; and
b) when said condition comprises myasthenia gravis, said at least one cannabinoid is selected from the group consisting of CBD, THC, THCa and combinations thereof, and said primary terpene is selected from the group consisting of pinene, terpineol, eucalyptol, pulegone, and p- cymene.
